(19) Europäisches Patentamt
European Patent Office
Office européen des brevets

(11) Publication number: **0 105 664**
**B1**

(12) # EUROPEAN PATENT SPECIFICATION

(45) Date of publication of patent specification: **20.11.86**

(51) Int. Cl.$^4$: **C 07 D 207/34**

(21) Application number: **83305548.6**

(22) Date of filing: **21.09.83**

(54) **Process for the preparation of 3-carboxy-1-methylpyrrol-2-acetic-acid and alkali metal salts thereof.**

(30) Priority: **21.09.82 IT 2337082**

(43) Date of publication of application:
**18.04.84 Bulletin 84/16**

(45) Publication of the grant of the patent:
**20.11.86 Bulletin 86/47**

(84) Designated Contracting States:
**BE DE FR GB NL**

(56) References cited:
**EP-A-0 089 392**
**EP-A-0 089 393**
**EP-A-0 092 487**
**US-A-3 865 840**
**US-A-4 048 191**
**US-A-4 363 918**

**The file contains technical information submitted after the application was filed and not included in this specification**

(73) Proprietor: **Montedison S.p.A.**
**31, Foro Buonaparte**
**I-20121 Milan (IT)**

(72) Inventor: **Bottaccio, Giorgio**
**16 Via Manin**
**Novara (IT)**
Inventor: **Campolmi, Stefano**
**27 Via Andrea Costa**
**Novara (IT)**
Inventor: **Carletti, Vittorio**
**7 Largo Europa**
**Meda, Milano (IT)**
Inventor: **Marchi, Marcello**
**11 Viale Allegra**
**Novara (IT)**

(74) Representative: **Whalley, Kevin et al**
**Marks & Clerk 57/60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 105 664 B1

**Description**

The present invention relates to a process for the preparation of 3-carboxy-1-methylpyrrol-2-acetic acid having the following formula (I):

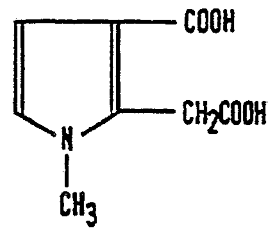

(I)

and alkali metal salts thereof.

More particularly, the present invention relates to a process for the preparation of 3-carboxy-1-methylpyrrol-2-acetic acid or an alkali metal salt thereof by condensation of a halo-acetaldehyde and of methylamine with acetone-bicarboxylic acid or an alkali metal salt thereof.

According to a particular aspect of the invention, alkali metal salts of 3-carboxy-1-methylpyrrol-2-acetic acid may be prepared according to above mentioned condensation reaction using an alkali metal salt of the acetone-bicarboxylic acid, preferably obtained in turn by the reaction of carbon dioxide and an alkali phenate with acetone.

The 3-carboxy-1-methylpyrrol-2-acetic acid obtained by the process of the invention is an important intermediate for the synthesis of organic compounds in general; more particularly it finds useful application in the preparation of compounds belonging to the class of 5-aryloyl-pyrrol-alkanoic acids, known in the pharmaceutical field as anti-inflammatory agents, and in particular 5-(p-methyl-benzoyl)-1-methyl-pyrrol-2-acetic acid of the formula (II):

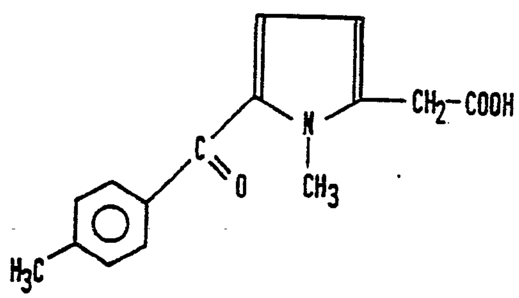

(II)

also known by the name "Tolmetin", as an effective anti-inflammatory agent.

It is known that compounds of the class of derivatives from 3-carboxy-1-alkylpyrrol-2-alkanoic acids are in general prepared in the form of esters. One process consists in reacting an alkylamine with chloroacetaldehyde and an alkyl ester of acetone-bicarboxylic acid in an aqueous reaction medium, at substantially room temperature, as disclosed for example in US—A—4048191.

This notwithstanding, the above specified process, for the purposes of the preparation of 5-aryloyl-pyrrol-alkanoic derivatives of greater interest, has the drawback of preparing such derivatives in the form of alkyl esters which, while on the one hand possessing a greater stability in comparison with salts or acids and thus being easier to store and transport, on the other hand require saponification both as regards general synthesis as well as, in particular, their further use as pharmaceutical substances as acids.

Moreover, the alkyl esters of acetone-bicarboxylic acid, used according to the above mentioned prior art process, are relatively expensive, while in addition they are intended to be saponified in the end product. This latter operation is economically and operationally disadvantageous in comparison with the remainder of the process for obtaining the 5-aryloxy-pyrrol-alkanoic acid derivative of main interest.

Thus, the present invention aims to provide a process for the prepartion of 3-carboxy-1-methylpyrrol-2-acetic acid of formula (I) as previously defined, and alkali metal salts thereof, which is simple and economical and free of the drawbacks of the previously discussed prior art processes.

A particular aim of the invention is that of providing a process for the preparation of alkali metal salts of 3-carboxy-1-methylpyrrol-2-acetic acid of formula (I), according to a method which is conveniently combinable with already available technology to allow the preparation of the alkali metal salt of the acid of formula (I) starting from acetone, carbon dioxide and the alkali phenate, raw materials that are particularly inexpensive.

The present invention provides a process for the preparation of 3-carboxy-1-methylpyrrol-2-acetic acid of formula (I), as previously defined, and alkali metal salts thereof, wherein acetone-bicarboxylic acid or an alkali metal salt thereof is reacted, in an aqueous medium, with methylamine and a halo-acetaldehyde, preferably chloroacetaldehyde, at a temperature from about 5°C to about 25°C.

The process according to the present invention preferably comprises admixing the acetone-bicarboxylic acid or the alkali metal salt thereof with methylamine in an aqueous medium at a temperature preferably from about 5°C to about 15°C, and by then gradually admixing in the same reaction medium chloroacetaldehyde in an aqueous solution, at a temperature that may be lower than about 15°C, and then carrying out the reaction up to its completion at room temperature.

This process may be schematically represented by the following equation:

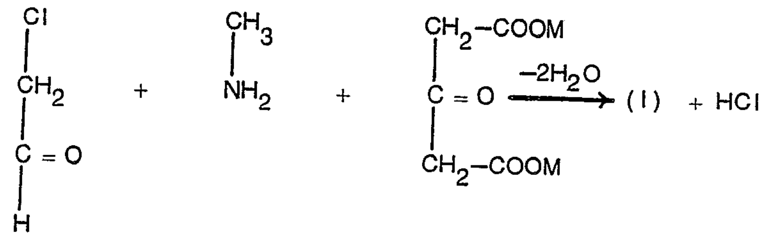


In above equation chloroacetaldehyde is used as the halo-acetaldehyde; similar results are achievable when using bromo-acetaldehyde. 'M' represents either a hydrogen or an alkali metal atom, preferably Na or K; in this latter case acid (I) is obtained in the form of its corresponding alkali metal salt.

The reactants are preferably introduced into the reactor according to substantially equimolar ratios, according to the stoichiometry of the above reaction.

The chloroacetaldehyde is preferably introduced in the form of one of its aqueous solutions, as commonly available.

In the case in which M = H, that is when acetone-bicarboxylic acid is employed, in order to satisfy the free acid functions there is required an excess of methylamine.

During the reaction the temperature is generally maintained below about 25°C. Better results are achieved, as previously indicated, by bringing the temperature gradually from about the initial range of 5°—15°C, at the moment of the addition of the methylamine to the acetone-bicarboxylic acid or to its aqueous alkali metal salt, up to a temperature below about 15°C, during the addition of the chloroacetaldehyde, and by then proceeding at room temperature.

Since the reaction is exothermic, the control of the reaction temperature is facilitated if the addition of the chloroacetaldehyde is carried out in a gradual way, under external cooling of the reactor.

The reaction times depend on the adopted conditions, but are preferably from about 2 to about 3 hours, sufficient for the completion of the reaction itself.

At the end of the reaction the product is separated and purified according to substantially conventional techniques.

For instance, at the end of the reaction, the reaction mixture may be poured in the cold state (less than 20°C) into an aqueous solution of hydrochloric acid, until a pH from 1 to 2 is reached, and the solid is then separated and washed with water and finally dried. By crystallization from acetic acid, washing with ethyl ether and subsequent drying, there is obtained a pure product.

According to a particularly preferred embodiment of the invention which is also advantageous from the economic point of view, the process of the invention may be directly applied to the alkali metal salts of acetone-bicarboxylic acid, obtained according to the known process for their preparation starting from acetone, carbon dioxide and alkali phenates, in the following way.

To a solution of alkali phenate in a suitable aprotic solvent, preliminarily saturated with carbon dioxide, under stirring there is added, at the pre-established temperature, the acetone in the established ratio; the reaction mixture is thereupon subjected to stirring under a carbon dioxide atmosphere until completion of the reaction. At this point the mixture is additioned with water in the pre-established quantity.

The alkali salts of the acetoacetic acid and 3-ketoglutaric acid produced, together with the biocarbonate derived from the excess of phenate, precipitate in a crystalline form and are then filtered and washed with a solvent (benzene, petroleum ether, chloroform).

The acid may be freed from the salt by known procedures, by acidification with dilute sulphuric acid followed by extraction with ethyl ether, etc.

The process according to the present invention may be suitably conducted in the following way.

Into a reactor provided with temperature-regulating systems and metering devices for dosing the reactants, there is introduced an aqueous solution of methylamine. To this mixture is then admixed the acetone-carboxylic acid at a temperature from 5° to about 15°C, or alternatively the raw mixture of the alkali metal salt of the acetone-carboxylic acid, as obtained according to the process of carboxylation of acetone with a phenate as described above.

To the mixture thus obtained there is added the chloroacetaldehyde in a 50% by weight aqueous solution, in a gradual way and maintaining the temperature below 15°C; thereafter the reaction is carried on at substantially room temperature for about 2 hours. At the end of the reaction the 3-carboxy-1-methyl-pyrrol-2-acetic acid or alkali metal salt produced is separated and purified as indicated above.

Because of the simple and mild operational conditions, the process is particularly convenient.

A particular advantage is in the possibility of utilizing directly the raw mixture containing the alkali

metal salt of the acetone-bicarboxylic acid, as produced in the process of carboxylation of acetone with $CO_2$ and alkali phenate. Although the raw mixture contains other alkali salts of organic acids (acetoacetates) and inorganic acids (carbonates and bicarbonates), the results obtained are practically unaffected by these impurities.

The invention will be further described with reference to the following illustrative Examples.

Example 1

Into a 250 cc flask provided with a stirrer, a thermometer, a dripping funnel and a loading system for the reactants, there were introduced 15 cc of water and 45 cc of a 35% by weight aqueous solution of methylamine. To this mixture were then added 7.3 g of acetone-bicarboxylic acid at a temperature of about 10°C. Thereupon into the mixture there were introduced dropwise over a time period of 10 minutes 11 cc of a 50% by weight aqueous solution of chloroacetaldehyde, keeping the temperature between 10°C and 15°C. The mixture was then kept under stirring for two hours at room temperature.

To this reaction mixture was then added dilute hydrochloric acid (at a concentration of about 10%), until a pH value of 1 was reached, while maintaining the temperature below 20°C.

There precipitated a solid which was filtered, washed with water and then dried. Thereby there were obtained 6.7 g of product which were crystallized by dissolving in about 100 cc of icy acetic acid at boiling point, by then cooling down to room temperature and then filtering the precipitated solid which was finally washed with ethyl ether and then dried.

There were finally obtained about 5 grams of a white product (m.p. 220°C, by sublimation) which was identified as 3-carboxy-1-methylpyrrol-2-acetic acid.

Example 2

43 grams of filtration cake, containing about 7 g of acetone-bicarboxylic acid in the form of bipotassic salt, obtained by the carboxylation reaction of the acetone with $CO_2$ and potassium 2,6-diterbutyl-p-cresolate in toluene, according to the operational procedure described in "Gazzetta Chimica Italiana" — 107, *1977*, page 499, were suspended in 20 cc of water, in a 250 cc reactor fitted with a stirrer, a thermometer, a dripping funnel and a loading system for the charging of the reactants.

Maintaining the temperature below 5°C, there were slowly dripped into the mixture 20 cc of $H_2SO_4$ at 60% concentration until a pH value equal to about 2 was reached. Thereupon, at a controlled temperature of about 15°C, into the mixture were introduced 45 cc of a aqueous 35% by weight solution of methylamine, followed by the addition, at the same temperature, of a 50% by weight aqueous soution of chloroacetaldehyde, over a time period of 10 minutes.

This reaction mixture was then maintained under stirring for 2 hours at room temperature. The suspension of salts was then filtered and the cake washed with 20 cc of water. The resulting filtrate was thereupon brought to a pH value of 1 by the addition of a 60% $H_2SO_4$, maintaining the temperature below 20°C.

At the end of this operation there precipitated a solid which was filtered, washed with water and dried. 5.6 grams of product thus obtained were crystallized from icy acetic acid, thereby obtaining 3.5 g of 3-carboxy-1-methylpyrrol-2-acetic acid (m.p. 220°C, by sublimation).

**Claims**

1. A process for the preparation of 3-carboxy-1-methylpyrrol-2-acetic acid of formula (I):

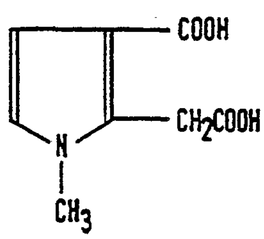

(I)

and alkali metal salts thereof, characterized in that acetone-bicarboxylic acid or an alkali metal salt thereof is reacted with methylamine and a haloacetaldehyde in an aqueous medium, at a temperature from about 5°C to about 25°C.

2. A process as claimed in Claim 1, characterized in that the alkali metal salt of acetone-bicarboxylic acid is a sodium or potassium salt thereof.

3. A process as claimed in Claim 2, characterized in that the said alkaki metal salt is a sodium or potassium salt of raw acetone-bicarboxylic acid obtained by the reaction of acetone with carbon dioxide and an alkali phenate in an organic medium.

4. A process as claimed in any of claims 1 to 3, characterized in that the said reactants are reacted in substantially stoichiometric amounts.

5. A process as claimed in any of Claims 1 to 4, characterized in that the reaction is conducted by admixing the methylamine with the acetone-bicarboxylic acid or its alkali metal salt at a temperature from about 5°C to about 15°C, and by then adding the haloacetaldehyde, and by then completing the reaction at room temperature.

6. A process as claimed in any of Claims 1 to 5, characterized in that the reaction is carried out over a time period of from about 2 to about 3 hours.

7. A process as claimed in any of Claims 1 to 6, characterized in that the halo-acetaldehyde is chloroacetaldehyde.

## Patentansprüche

1. Verfahren zur Herstellung von 3-Carboxy-1-methylpyrrol-2-essigsäure der Formel (I)

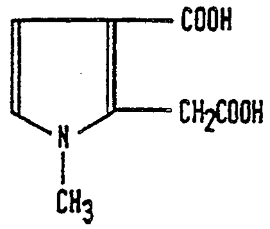

(I)

und deren Alkalimetallsalzen, dadurch gekennzeichnet, daß Acetonbicarbonsäure oder ein Alkalimetallsalz derselben mit Methylamin und einem Halogenacetaldehyd in einem wässrigen Medium bei einer Temperatur von etwa 5 bis etwa 25°C umgesetzt wird.

2. Verfahren nach Anspruch 1, dadurch gekennzeichnet, daß das Alkalimetallsalz der Acetonbicarbonsäure ein Natrium- oder Kaliumsalz derselben ist.

3. Verfahren nach Anspruch 2, dadurch gekennzeichnet, daß das Alkalimetallsalz ein Natrium- oder Kaliumsalz einer rohen Acetonbicarbonsäure, erhalten durch die Reaktion von Aceton mit Kohlendioxid und einem Alkaliphenolat in einem organischen Medium, ist.

4. Verfahren nach einem der Ansprüche 1 bis 3, dadurch gekennzeichnet, daß die Reaktionsteilnehmer in praktisch stöchiometrischen Mengen umgesetzt werden.

5. Verfahren nach einem der Ansprüche 1 bis 5, dadurch gekennzeichnet, daß die Reaktion durch Mischen des Methylamins mit der Acetonbicarbonsäure oder deren Alkalimetallsalz bei einer Temperatur von etwa 5 bis etwa 15°C und anschließender Zugabe des Halogenacetaldehyds sowie nachfolgender Beendigung der Reaktion bei Raumtemperatur erfolgt.

6. Verfahren nach einem der Ansprüche 1 bis 5, dadurch gekennzeichnet, daß die Reaktion über eine Dauer von etwa 2 bis etwa 3 h erfolgt.

7. Verfahren nach einem der Ansprüche 1 bis 6, dadurch gekennzeichnet, daß der Halogenacetaldehyd Chloracetaldehyd ist.

## Revendications

1. Procédé de préparation de l'acide 3-carboxy-1-méthylpyrrol-2-acétique de formule (I):

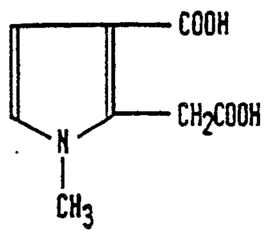

(I)

et de ses sels de métaux alcalins, caractérisé en ce que l'on fait réagir l'acide acétone bicarboxylique ou un de ses sels de métal alcalin avec la méthylamine et un haloacétaldéhyde en milieu aqueux à une température d'environ 5°C à environ 25°C.

2. Procédé selon la revendication 1, caractérisé en ce que le sel de métal alcalin de l'acide acétone-bicarboxylique est son sel de sodium ou de potassium.

3. Procédé selon la revendication 2, caractérisé en ce que ce sel de métal alcalin est un sel de sodium ou de potassium de l'acide acétone bicarboxylique brut obtenu par la réaction de l'acétone avec le dioxyde de carbone et un phénolate alcalin en milieu organique.

4. Procédé selon l'une quelconque des revendications 1 à caractérisé en ce que l'on fait réagir ces réactifs en quantités sensiblement stoéchiométriques.

5. Procédé selon l'une quelconque des revendications 1 à 4, caractérisé en ce que l'on conduit la réaction en mélangeant la méthylamine avec l'acide acétone-bicarboxylique ou son sel de métal alcalin à une température d'environ 5°C à environ 15°C et ensuite en ajoutant l'haloacétaldéhyde et en achevant, ensuite, la réaction à la température ambiante.

6. Procédé selon l'une quelconque des revendications 1 à 5, caractérisé en ce que l'on effectue la réaction sur une période de temps d'environ deux à environ trois heures.

7. Procédé selon l'une quelconque des revendications 1 à 6, caractérisé en ce que l'haloacétaldéhyde est le chloroacétaldéhyde.